# EUROPEAN PATENT SPECIFICATION

(11) **EP 1 351 668 B1**
(45) Date of publication and mention of the grant of the patent: **15.10.2014**
(21) Application number: 01998126.5
(22) Date of filing: 20.12.2001
(51) Int. Cl.: A61K 9/22

(54) **SUSTAINED RELEASE PHARMACEUTICAL DOSAGE FORMS WITH MINIMIZED PH DEPENDENT DISSOLUTION PROFILES**
PHARMAZEUTISCHE RETARDDOSIERUNGSFORMEN MIT MINIMIERTEN PH-ABHÄNGIGEN AUFLÖSUNGSVERLÄUFEN
FORMES DE DOSAGE PHARMACEUTIQUE A LIBERATION PROLONGEE POSSEDANT DES PROFILES DE DISSOLUTION A DEPENDANCE AU PH REDUITE AU MINIMUM

(30) Priority: 20.12.2000 US 741548
(43) Date of publication of application: 15.10.2003
(73) Proprietor: Supernus Pharmaceuticals, Inc., Rockville, MD 20850 (US)
(72) Inventor: BURNSIDE, Beth, Brookeville, MD 20833 (US); CHANG, Rong-Kun, Rockville, MD 20850 (US); GUO, Xiaodi, New City, NY 10956 (US)
(74) Representative: Walcher, Armin
(86) International application number: PCT/US2001/050773
(87) International publication number: WO 2002/058676

(56) References cited:
- EP-A1- 0 320 097
- EP-A1- 0 373 417
- WO-A1-99/21551
- WO-A2-00/41681
- WO-A2-99/20223
- US-A- 4 968 508
- US-A- 5 102 668
- US-A- 5 229 135
- US-A- 5 378 474

## Description

This invention relates to pharmaceutical compositions. More particularly, this invention relates to pharmaceutical compositions having a pH-independent or a minimized pH-dependent dissolution profile. In particular, such composition includes at least one pharmaceutically active agent that has a pH dependent solubility profile, at least one non-pH-dependent sustained release agent, and at least one pH-dependent agent that increases the dissolution rate of the at least one pharmaceutically active agent at a pH in excess of 5.5. The active agent(s) has (have) a solubility profile wherein the active agent(s) is (are) more soluble in an acidic medium than in a basic medium.

The rate at which a drug goes into solution when it is dissolved in a medium is proportional to the solubility of the drug in the medium. Many drugs have different solubilities at different pHs. These pH-dependent solubility differences lead to pH-dependent dissolution profiles. In general, pH-dependent dissolution is an undesirable product characteristic.

Compressed matrix tablets containing a basic drug often give a faster dissolution profile in simulated gastric fluid, having a pH about 1.0, than in simulated intestinal fluid (pH 6.8 to 7.4).

WO 99/21551 A1 discloses a matrix for controlled delivery of highly soluble drugs.

EP 0 320 097 A1 discloses controlled diltiazem formulations.

WO 99/66916 A1 discloses apomorphine-containing dosage forms for ameliorating male erectile dysfunction.

It is an object of the present invention to provide a pharmaceutical composition with a minimized pH dependent or a pH-independent dissolution profile.

In accordance with an aspect of the present invention, there is provided a pharmaceutical composition according to claim 1.

Pharmaceutically active agents which are pH dependent and which are included in the composition include weakly basic drugs and their salts that have higher solubilities at lower pH levels. Such drugs include guanfacine hydrochloride, guanadrel sulfate, reserpine, anagrelide hydrochloride, propranolol, metoprolol, atenolol, timolol, erthyrthromycin, clonidine, chlorpheniramine, bromopheniramine, diltiazen, and scopolamine. In general, the pharmaceutically active agent is present in the composition in an amount of from about 0.1 wt, % to about 70 wt. %, preferably from about 1 wt. % to about 40 wt %. In one embodiment, the at least one pharmaceutically active agent is guanfacine hydrochloride. In another embodiment, the at least one pharmaceutically active agent is anagrelide hydrocholoride.

Non-pH-dependent sustained release agents which may be included in the composition include, but are not limited to, ethylcellulose, cellulose acetate, vinyl acetate/vinyl chloride copolymers, acrylate/methacrylate copolymers, polyethylene oxide, hydroxypropyl methylcellulose, hydroxyethyl cellulose, hydroxypropyl cellulose, karaya gum, acacia gum, tragacanth gum, locust bean gum, guar gum, methyl cellulose, beeswax, carnauba wax, cetyl alcohol, hydrogenated vegetable oils, and stearyl alcohol. In general, the at least one non-pH-dependent sustained release agent is present in the composition in an amount of from about 5 wt. % to about 50 wt. %, preferably from about 10 wt. % to about 30 wt. %. It is to be understood, however, that the scope of the present invention is not to be limited to any particular non-pH- dependent sustained release agents.

pH-dependent agents that increase the rate of release of the at least one pharmaceutically active agent from the tablet at a pH in excess of 5.5 include, but are not limited to, polymers that swell at a pH in excess of 5.5, and enteric agents, and/or agents that increase the solubility of the at least one pharmaceutically active agent at a pH greater than 5.5, by maintaining an acidic microenvironment in the tablet, e.g., an organic acid. The at least one pH-dependent agent is present in the composition in an amount of from about 0.5 wt. % to about 40 wt. %, preferably from about 1 wt. % to about 20 wt. %.

Polymers that swell at a pH in excess of 5.5 include, but are not limited to, acrylic acid copolymers, sodium alginate, carrageenan, alginic acid, pectin, and sodium carboxymethyl cellulose.

Enteric agents include, but are not limited to, cellulose acetate phthalate, hydroxypropyl methylcellulose phthalate, polyvinyl acetate phthalate, methacrylic acid coplymers, cellulose acetate trimellitate, hydroxypropyl methylcellulose acetate, succinate, shellac, and zein.

Agents that increase the solubility of the at least one pharmaceutically active agent at a pH greater than 5.5 include, but are not limited to, organic acids. Such organic acids maintain an acidic microenvironment in the tablet, and include, but are not limited to, citric acid, fumaric acid, tartaric acid, adipic acid, glucono delta-lactone, and malic acid.

The composition of the present invention may further include other materials such as bulking agents, disintegrating agents, anti-adherants and glidants, lubricants, and binding agents.

Bulking agents include, but are not limited to, microcrystalline cellulose (eg., Avicel®, FMC Corp., Emcocel®, Mendell Inc.), mannitol, xylitol, dicalcium phosphate (eg. Emcompress, Mendell Inc.) calcium sulfate (eg. Compactrol, Mendell Inc.) starches, lactose, sucrose (Dipac, Amstar, and Nutab, Ingredient Technology), dextrose (Emdex, Mendell, Inc.), sorbitol, cellulose powder (Elcema, Degussa, and Solka Floc, Mendell, Inc.) The bulking agent may be present in the composition in an amount of from about 5 wt. % to about 90 wt. %, preferably from about 10 wt. % to about 50 wt. %.

Disintegrating agents which may be included in the composition include, but are not limited to, microcrystalline cellulose, starches, crospovidone (eg. Polyplasdone XL, International Specialty Products.), sodium starch glycolate (Explotab, Mendell Inc.), and crosscarmellose sodium (eg., Ac-Di-Sol, FMC Corp.). The disintegrating agent may be present in the composition in an amount of from about 0.5 wt. % to about 30 wt %, preferably from about 1 wt. % to about 15wt.%.

Antiadherants and glidants which may be employed in the composition include, but are not limited to, talc, corn starch, silicon dioxide, sodium lauryl sulfate, and metallic stearates.

The antiadherant or glidant may be present in the composition in an amount of from about 0.2 wt. % to about 15 wt. %, preferably from about 0.5 wt. % to about 5 wt. %.

Lubricants which may be employed in the composition include, but are not limited to, magnesium stearate, calcium stearate, sodium stearate, stearic acid, sodium stearyl fumarate, hydrogenated cotton seed oil (sterotex), talc, and waxes, including but not limited to, beeswax, carnuba wax, cetyl alcohol, glyceryl stearate, glyceryl palmitate, glyceryl behenate, hydrogenated vegetable oils, and stearyl alcohol. The lubricant may be present in an amount of from about 0.2 wt. % to about 20 wt. %, preferably from about 0.5 wt. % to about 5 wt. %.

Binding agents which may be employed include, but are not limited to, polyvinyl pyrrollidone, starch, methylcellulose, hydroxypropyl methylcellulose, carboxymethyl cellulose, sucrose solution, dextrose solution, acacia, tragacanth and locust bean gum. The binding agent may be present in the composition in an amount of from about 0.2 wt. % to about 10 wt. %, preferably from about 0.5 wt. % to about 5 wt. %.

The compositions of the present invention may be made by a direct compression method, or by a wet granulation method. In the direct compression method, the at least one pharmaceutically active agent and other ingredients are sieved through a stainless steel screen, such as a 40 mesh steel screen. The sieved materials then are charged to a suitable blender, and blended for 10 minutes with an intensifier bar on for 3 minutes. The blend then is compressed into tablets on a rotary press using appropriate tooling. The compressed tablets may be coated, if desired.

In the wet granulation method, the at least one pharmaceutically active agent and other ingredients are granulated with a granulating fluid (e.g., isopropyl alcohol, ethyl alcohol, and water) in a planetary mixer, high shear mixer, or fluidized bed granulator. Binding agents may be contained in the granulating fluid, or may be in the dry mix. The wet granules are dried in an oven or fluidized bed dryer, and then sieved through a suitable screen to obtain free flowing granules. The resulting granules were blended with a suitable lubricant and glidant, and the lubricated granules are compressed into tablets on a rotary press using appropriate tooling. If desired, a coating can be applied onto the compressed tablets.

When the pharmaceutically active agent is guanfacine hydrochloride, the composition may be employed in treating an attention deficit disorder, or attention deficit with hyperactivity disorder. The composition including guanfacine hydrochloride is administered to an animal, such as a mammal, including human and non-human primates, in an amount effective to treat the disorders mentioned hereinabove.

The compositions of the present invention may be employed to treat a variety of diseases or disorders.

When guanfacine hydrochloride is administered as part of a composition in accordance with the present invention, there is a reduction in the number of side effects associated with the administration of guanfacine hydrochloride, or a reduction in the likelihood of side effects associated with the administration of guanfacine hydrochloride.

In accordance with a further aspect of the present invention there is provided a pharmaceutical composition comprising guafancine as an active agent and characterized in that said composition when administered to a patient produces less of the side effects usually associated with guafancine.

In one embodiment the side effects are chosen from Headache, Asthenia, Somnolence , Dizziness, Tacchycardia, Nausea, Pain Abdominal, Infect, Vomit, or Palpitations.

In a further embodiment the side effects are chosen from Headache or Asthenia.

When the pharmaceutically active agent is anagrelide, the composition may be employed in treating a variety of blood disorders, including, but not limited to, myeloproliferative blood disorders or MBDs, such as, for example, essential thrombocythemia, or ET, chronic myelogenous leukemia, or CML, polycythemia vera, or PV, and agnogenic myeloid metaplasia, or AMM. The composition including anagrelide may be administered to an animal, such as a mammal, including human and non-human primates, in an amount effective to treat such disorders.

The active agent anagrelide may be administered to a patient in an amount of from about 0.01 mg to 15 mg, preferably from about 0.1 mg to about 10 mg, more preferably from about 0.1 mg to about 5 mg and most preferably from about 0.5 mg to about 2 mg. In one treatment regimen, anagrelide may be administered to a patient in an amount of 0.5 mg four times a day, or 1 mg twice a day, for at least one week. When anagrelide is administered as part of a composition in accordance with the present invention, there is a reduction in the number of side effects associated with the administration of anagrelide, or a reduction of the likelihood of side effects associated with the administration of anagrelide.

In accordance with a further aspect of the present invention there is provided a pharmaceutical composition comprising anagrelide as an active agent and characterized in that said composition when administered to a patient produces less of the side effects usually associated with anagrelide.

In one embodiment the side effects are chosen from Headache, Asthenia, Somnolence , Dizziness, Tacchycardia, Nausea, Pain Abdominal, Infect, Vomit, or Palpitations.

In a further embodiment the side effects are chosen from Headache or Asthenia.

The invention now will be described with respect to the following examples; however, the scope of the present invention is not intended to be limited thereby.

### Example 1

Formulations in accordance with the present invention, as well as control formulations, were prepared according to the direct compression described hereinabove. In accordance with such method, guanfacine HCl and other ingredients were sieved through a 40 mesh steel screen. The sieved materials then were charged into a Blendmaster blender (Patterson-Kelley Co.), and blended for 10 minutes with an intensifier bar on for 3 minutes. The blends then are compressed into tablets on a rotary tablet press (Stokes-Merrill Corp., Model 512) using appropriate tooling The formulations are given in Table 1 below

**Table 1**

| | **PD0052-22A** | | **PD0052-25B** | | **PD0052-28B** | | **PD0052-32B** | | **PD0052-32D** | | **PD0052-39A** | |
|---|---|---|---|---|---|---|---|---|---|---|---|---|
| **Ingredient** | 1* | 2** | 1 | 2 | 1 | 2 | 1 | 2 | 1 | 2 | 1 | 2 |
| **Guanfacine HCl** | 0.57 | 1.14 | 0.57 | 1.14 | 0.57 | 1.14 | 0.57 | 1.14 | 0.57 | 1.14 | 0.57 | 1.1 4 |
| **Lactose** | 37.43 | 74.86 -- | -- | -- | -- | -- | 45.00 | 90.00 | 65.00 | 130.0 -- | -- | -- |
| **ProSolv** | 20.00 | 40.00 | -- | -- | 30.00 | 60.00 | -- | -- | -- | -- | 29.82 | 59.64 |
| **Polyox WSR** | 40.00 | 80.00 | 40.00 | 80.00 | -- | -- | 40.00 | 80.00 | 10.00 | 20.00 | | |
| **Ethocel FP** | -- | -- | -- | -- | 40.00 | 80.00 | -- | -- | -- | -- | 39.82 | 79.64 |
| **Cellulose acetate** | -- | -- | 50.00 | 100.00 | -- | -- | -- | -- | -- | -- | -- | -- |
| **Sodium alginate** | -- | -- | -- | -- | 20.00 | 40.00 | -- | -- | -- | -- | -- | -- |
| **Carrageenan** | -- | -- | -- | -- | -- | -- | -- | -- | -- | -- | 19.91 | 39.82 |
| **Carbopol 974P** | -- | -- | -- | -- | -- | -- | -- | -- | 10.00 | 20.00 | -- | -- |
| **Fumaric acid** | -- | -- | -- | -- | -- | -- | 5.00 | 10.00 | -- | -- | -- | -- |
| **Eudragit L100-55** | -- | -- | -- | -- | -- | -- | -- | -- | 5.00 | 10.00 | -- | -- |
| **EDTA** | -- | -- | -- | -- | -- | -- | -- | -- | -- | -- | 0.50 | 1.00 |
| **Compritol** | -- | -- | 9.43 | 18.86 | 9.43 | 18.63 | 9.43 | 18.63 | 9.43 | 18.63 | 9.37 | 18.74 |
| **Stearic acid** | 2.00 | 4.00 | -- | -- | -- | -- | -- | -- | -- | -- | -- | --- |

| | | | | | | | | | | | | |
|---|---|---|---|---|---|---|---|---|---|---|---|---|
| * 1 = composition in % weight **2 = composition in mg per tablet Note: - PD0052-22A and PD0052-25B contain no ionic materials in the formulations. These two formulations serve as a control. - Prosolv is a trade name for silicified microcrystalline cellulose and marketed by Penwest Corp. - Polyox is a trade name for poly(ethyleneoxide) and marketed by Union Carbide. - Carbopol is a trade name for copolymer of acrylic acid and marketed by BF Goodrich. - Ethocel FP is a trade name for ethyl cellulose fine powder grade and marketed by Dow Chemical. - Eudragit L100-55 is a trade name for poly(methacrylic acid, ethyl acrylate) and marketed by Rohm GmbH. - Compritol is a trade name for glyceryl behenate and marketed by Gattefosse. | | | | | | | | | | | | |

The dissolution data was determined as follows:
A Vankel dissolution tester (VanKel Industries, Edison, N.J.) was used for all dissolution studies. The apparatus was calibrated according to USP23. The dissolution in 0.1N hydrochloric acid (pH 1.2) or pH 6.8 phosphate buffer was tested using the paddle method (USP Apparatus II), employing 500 ml of dissolution medium at a temperature of 37°C and an agitation rate of 50 rpm. Samples at specific time points were removed and filtered through a 35 µm filter. The filtered samples were kept in screw cap glass test tubes until analysis. An HPLC system comprised of an autosampler and a pump and a UV detector was used for sample analysis. 50µl of the dissolution samples were injected directly on the HPLC C18 column using a mixture of acetonitrile and acetate buffer (20:80) as the mobile phase.

The dissolution data are given in Table 2 below.

**TABLE 2**

| **Dissolution Data for Guanfacine Sustained Release Tablets** | | | | | | | | | | | | |
|---|---|---|---|---|---|---|---|---|---|---|---|---|
| | **PD0052-22A** | | **PD0052-25B** | | **PD0052-28B** | | **PD0052-32B** | | **PD0052-32D** | | **PD0052-39A** | |
| Time(hour) | 1* | 2** | 1 | 2 | 1 | 2 | 1 | 2 | 1 | 2 | 1 | 2 |
| 0.5 | 14.0± 0.6 | 7.0± 1.2 | 13.0 ± 1.5 | 5.0 ± 0.6 | 8.4 ± 2.1 | 10.0 ± 0.6 | 19.0 ± 1.2 | 2.0 ± 0.0 | 1.0 ± 1.2 | 11.0 ± 1.0 | 14.0 ± 0.6 | 10.0 ± 1.0 |
| 1.0 | 24.0 ± 1.0 | 12.0 ± 1.0 | 27.0 ± 2.0 | 7.0 ± 1.0 | 41.0 ± 1.7 | 20.0 ± 1.0 | 31.0 ± 2.6 | 20.0 ± 0.6 | 48.0 ± 1.2 | 19.0 ±0.6 | 25.0 ± 1.0 | 18.0 ± 2.0 |
| 2.0 | 44.0 ± 0.6 | 19.0 ± 1.5 | 48.0 ± 2.5 | 11.0 ± 0.6 | 64.0 ± 2.5 | 36.0 ± 2.5 | 50.0 ± 4.2 | 35.0 + 0.6 | 80.0 ± 5.6 | 31.0 ± 1.2 | 42.0 ± 1.0 | 31.0 + 4.0 |
| 3.0 | 59.0 ±0.6 | 26.0 ± 1.5 | 63.0 ± 1.7 | 14.0 ± 0.6 | 77.0 ± 2.6 | 49.0 ± 3.6 | 65.0 ± 6.1 | 49.0 + 1.0 | 96.0 ± 5.0 | 46.0 ± 0.6 | 56.0 ± 2.6 | 46.0 ± 4.4 |
| 4.0 | 71.0 ±6.0 | 31.0 ±2.9 | 74.0 ±1.5 | 16.0 ± 0.6 | 86.0 ± 3.2 | 59.0 ± 3.2 | 77.0 ± 5.5 | 61.0 ± 1.0 | 104 ± 3.5 | 56.0 ± 0.6 | 69.0 ± 3.0 | 56.0 ± 5.8 |
| 6.0 | 90.0 ± 2.0 | 39.0 + 2.5 | 86.0 ± 1.7 | 19.0 ± 0.0 | 97.0 ± 3.5 | 71.0 ± 5.0 | 99.0 ± 4.4 | 87.0 ± 2.5 | 111 ± 4.0 | 74.0 ± 1.2 | 90.0 ± 2.6 | 72.0 ± 6.2 |
| 8.0 | 99.0 ± 1.7 | 46.0 ± 2.5 | 93.0 ± 2.1 | 21.0 ± 1.0 | 108 ± 3.8 | 80.0 ± 5.5 | 102 ± 1.2 | 97.0 ± 1.5 | 112 +4.0 | 89.0 ± 2.3 | 102 ± 2.3 | 83.0 ± 6.4 |
| 12.00 | 105.0 + 1.5 | 61.0 ± 4.7 | 100.0 ± 1.2 | 25.0 ± 1.0 | 113 ± 3.6 | 91.0 ± 6.0 | 103 ± 2.3 | 98.0 ± 1.0 | 113 ± 3.8 | 107 ± 2.9 | 112 ±0.6 | 96.0 ±4.2 |

| | | | | | | | | | | | | |
|---|---|---|---|---|---|---|---|---|---|---|---|---|
| * 1 = percent dissolved using a pH 1.2 dissolution medium **2= percent dissolved using a pH 6.8 dissolution medium Note: The data represent the mean percent dissolved ± standard deviation of three replicate. | | | | | | | | | | | | |

The above results show that the compositions of the present invention have improved dissolution profiles when compared with the control compositions.

### Example 2

Formulations in accordance with the present invention, as well as control formulations, were prepared according to the direct compression method described hereinabove. In accordance with such method, guanfacine HCl and other ingredients were sieved through a 40 mesh steel screen. The sieved materials then were charged into a Blendmaster blender (Patterson-Kelley Co.), and blended for 10 minutes with an intensifier bar on for 3 minutes. The blends then are compressed into tablets on a rotary tablet press (Stokes-Merrill Corp., Model 512) using appropriate tooling

The formulations are given in Table 3 below.

**Table 3. Weight Percentage of Components in Guanfacine MR Tablets, 1 mg, Batch Numbers 2015.00.001, 2016.00.001 and 2018.00.001**

| **Component** | **2015.00.001 (%)** | **2016.00.001 (%)** | **2018.00.001 (%)** |
|---|---|---|---|
| Guanfacine HCl, USP | 0.57 | 0.57 | 0.57 |
| Hydroxypropryl Methylcellulose (Methocel K4M Premium CR), USP/NF | 10.00 | 15.00 | 5.00 |
| Methacrylic Acid Copolymer (Eudragit L 100-55), USP/NF | 25.00 | - | - |
| Ammonio Methacrylate Copolymer (Eudragit RSPO), USP/NF | - | 25.00 | 22.50 |
| Silicified Microcrystalline Cellulose (Prosolv HD 90) | 25.00 | 20.00 | 34.43 |
| Lactose Monohydrate Povidone Crospovidone Granulated Blend (Ludipress) | 24.43 | 24.43 | 20.00 |
| Fumaric Acid, USP/NF | 5.00 | 10.00 | 5.00 |
| Glyceryl Behenate (Compritol 888 ATO), USP/NF | 10.00 | 5.00 | 12.50 |
| **Total** | **100.00** | **100.00** | **100.00** |

### Example 3

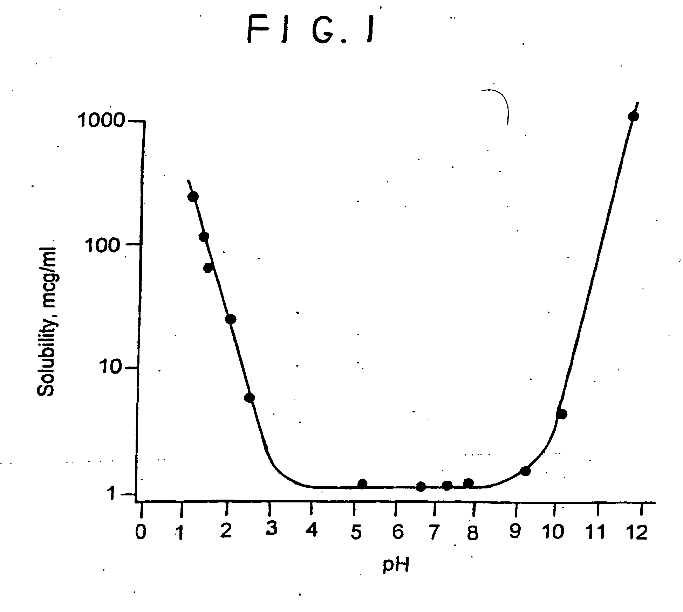

The solubility of anagrelide HCl in aqueous solutions in the pH range of 1 to 11.4 at 25°C was determined. The solubility-pH profile of anagrelide HCl is shown in Figure 1. Below pH 3, the solubility increased as the pH decreased which is consistent with formation of a more soluble protonated form. At pH 0.96 the solubility was 236 mcg/mL. Above pH 4, the solubility was independent of pH and remained constant (ca. 1.2 mcg/mL) up to pH 8. Above pH 8, the solubility increased with increasing pH which is due to the ionization of the quinazoline moiety. The solubility at pH 11.4 was 992 mcg/mL.

Formulations I through IV were formulated according to the procedure described in Example 1, except that anagrelide HCl has been substituted for guanfacine HCl.

The formulations are given in Table 4 below.

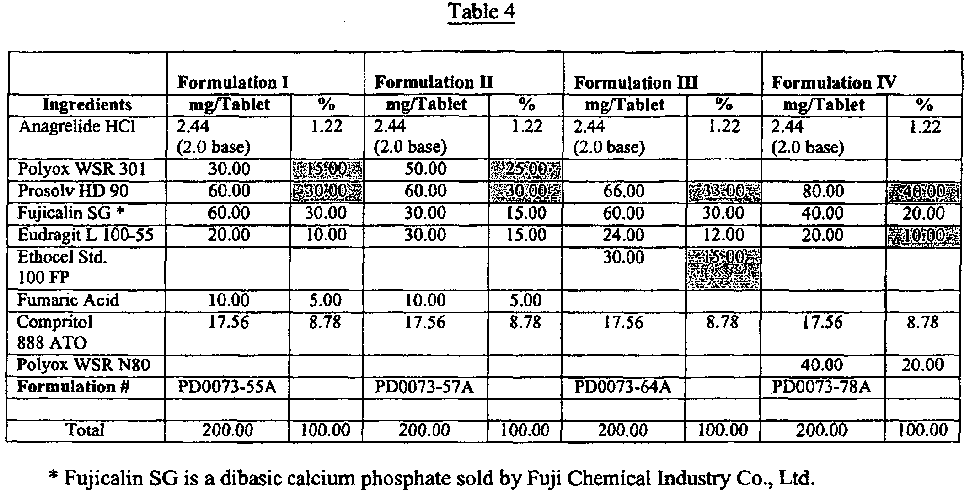

A Vankel dissolution tester (VanKel Industries, Edison, N.J.) was used for all dissolution studies. The apparatus was calibrated according to USP 23. The dissolution in 0.1N hydrochloric acid (pH 1.1) with 0.1% Tween 80 or pH 6.8 phosphate buffer with 0.1% Tween 80 was tested using the paddle method (USP Apparatus II), employing 900 ml of dissolution medium at a temperature of 37°C, and an agitation rate of 100 rpm. Samples at specific time points were removed and filtered through a 70µm filter. The filtered samples were kept in screw cap glass test tubes until analysis. An HPLC system composed of an autosampler and a pump and a UV detector was used for sample analysis. 20µl of the dissolution samples were injected directly on the HPLC C18 column using a mixture of acetonitrile and ammonium acetate buffer (36:64) as the mobile phase.

The dissolution data are given in Table 5 below.

**Table 5**

| | | | | | | | | |
|---|---|---|---|---|---|---|---|---|
| **Dissolution Data for Anagrelide Sustained Release Tablets** | | | | | | | | |

| | **PD0073-55A** | | **PD0073-57A** | | **PD0073-64A** | | **PD0073-78A** | |
|---|---|---|---|---|---|---|---|---|
| **Time (hour)** | **1*** | **2**** | **1** | **2** | **1** | **2** | **1** | **2** |
| **0.5** | N/A | 4.0± 0.6 | 6.0± 0.0 | 2.0± 0.0 | 27.0± 2.1 | 5.0± 1.0 | 16.0± 0.6 | 12.0± 1.2 |
| **1.0** | 13.0± 0.6 | 6.0± 0.0 | 9.0± 0.6 | 3.0± 0.0 | 39.0± 3.1 | 11.0± 2.9 | 27.0± 1.5 | 30.0± 1.0 |
| **2.0** | 21.0± 0.6 | 10.0± 0.6 | 15.0± 0.6 | 7.0± 0.0 | 52.0± 4.5 | 31.0± 3.2 | 43.0± 3.6 | 56.0± 1.7 |
| **4.0** | 40.0± 2.5 | 22.0± 1.2 | 30.0± 2.3 | 16.0± 0.6 | 69.0± 5.3 | 58.0± 3.5 | 55.0± 4.4 | 72.0± 1.2 |
| **8.0** | 72.0± 5.0 | 57.0± 7.4 | 57.0± 2.0 | 39.0± 1.0 | 85.0± 2.6 | 73.0± 2.6 | 67± 5.5 | 78.0± 0.6 |
| **12.0** | 95.0± 2.9 | 77.0± 5.3 | 77.0± 2.1 | 62.0± 3.6 | 88.0± 1.0 | 79.0± 2.1 | 83± 3.6 | 82.0± 0.0 |

| | | | | | | | | |
|---|---|---|---|---|---|---|---|---|
| *1=percent dissolved using a pH 1.1 dissolution medium with 0.1% Tween 80. **2=percent dissolved using a pH 6.8 dissolution medium with 0.1% Tween 80. Note: The data represent the mean percent dissolved ± standard deviation of three replicates. | | | | | | | | |

### Example 4

Formulations V, VI, and VII, which are modifications of Formulation IV of Example 2, include the following components as shown in Table 6 below.

**Table 6.**

| **Component** | **Formulation V** | | **Formulation VI** | | **Formulaiton VII** | |
|---|---|---|---|---|---|---|
| | **mg/ tablet** | **%** | **mg/ tablet** | **%** | **mg/ tablet** | **%** |
| Anagrelide HCl, Monohydrate * | 0.63 | 0.315 | 1.26 | 0.630 | 1.88 | 0.940 |
| Polyethylene Oxide, 60 Mesh, NF (Polyox™ WSR N80) | 40.00 | 20.000 | 40.00 | 20.000 | 40.00 | 20.000 |
| Dibasic Calcium Phosphate, Anhydrous, USP (Fujicalin SG) | 39.21 | 19.605 | 37.18 | 18.590 | 38.56 | 19.280 |
| Silicified High Density Microcrystalline Cellulose (Prosolv HD 90), USP | 80.00 | 40.000 | 80.00 | 40.000 | 80.00 | 40.000 |
| Methacrylic Acid Copolymer, NF (Eudragit L 100-55) | 20.00 | 10.000 | 20.00 | 10.000 | 20.00 | 10.000 |
| Glyceryl Behenate, NF (Compritol 888 ATO) | 17.56 | 8.780 | 17.56 | 8.780 | 17.56 | 8.780 |
| Magnesium Stearate Powder, NF (Non-Bovine) | 2.00 | 1.000 | 2.00 | 1.000 | 2.00 | 1.00 |
| Green PB-1763 | 0.60 | 0.300 | - | - | - | - |
| Purple PB-1855 | - | - | 2.00 | 1.000 | - | - |
| Total | **200.0** | **100.0** | **200.0** | **100.0** | **200.0** | **100.0** |

| | | | | | | |
|---|---|---|---|---|---|---|
| * includes 3% overcharge for material loss during processing. | | | | | | |

Formulations V, VI, and VII were prepared and processed into tablets according to the following procedure:

### Blending

1. Weigh all the ingredients (Prosolv HD90 is divided into 3 portions).
2. Place the ingredients in the order listed below into the V-blender equipped with an intensifier bar:
   a. Prosolv HD90 portion #1
   b. Anagrelide HCl Monohydrate
   c. Green PB-1763 for Formulation V; Purple PB-1855 for Formulation VI strength; no color for Formulation VII
   d. Prosolv HD90 portion #2
3. Blend for 4 minutes in a V-blender with the intensifier bar off. Turn the intensifier bar on and blend for 2 minutes. Turn the intensifier bar off and blend for 4 minutes.
4. Pass the blend through a 30-mesh screen using a Comil at the low speed setting.
5. Pass Prosolv HD90 portion #3 through the same Comil using the 30-mesh screen and low speed.
6. Place the ingredients into the larger V-blender equipped with an intensifier bar in the following order:
   a. Milled Prosolv HD90 from Step 5
   b. Polyox WSR N80, 60 mesh
   c. Milled blend from Step 4
   d. Eudragit L100-55
   e. Fujicalin SG
7. Blend these ingredients for 4 minutes with intensifier bar off. Turn the intensifier bar on and blend for 2 minutes. Turn the intensifier bar off, add Compritol 888 ATO and blend for 4 minutes.
8. Discharge this blend from the V-blender into a suitable container.
9. Pass Magnesium Stearate through a 30-mesh screen and collect it in a polyethylene bag.
10. Pass the blend from step 8 through a 30-mesh screen using a Comil at the low speed setting and collect it in a polyethylene bag.
11. Transfer the milled blend from step 10 and the Magnesium Stearate from step 9 into the V-blender used in step 7 and mix for 5 minutes with the intensifier bar off.
12. Discharge the blend from step 11 into a polyethylene bag.

### Tableting

1.Load the blend into the tablet press hopper.
2.Adjust the tablet weight to 200 mg and the appropriate tablet hardness.
3.Compress the blend into tablets using caplet-shaped tooling.
4.Take tablet samples as required by departmental SOPs to ensure product quality and to complete any process protocols of the tableting process.
5.Run tablets through an appropriate deduster.
6.Collect compressed tablets in a suitable container double lined with clean polyethylene bags.

### Example 5

Formulations 1, 2, and 3, were prepared, which had the following components in the following amounts, as shown in Table 7 below.

**TABLE 7**

| | **Amount, wt %** | | |
|---|---|---|---|
| **Component** | **Formulation 1** | **Formulation 2** | **Formulation 3** |
| Anagrelide HCl, Monohydrate | 0.61 | 0.61 | 0.61 |
| Polyethylene Oxide | 15.00 | -- | 25.00 |
| (Polyox™ WSR 301) | | | |
| Polyethylene Oxide, 60 Mesh, NF | -- | 20.00 | -- |
| (Polyox™ WSR N80) | | | |
| Dibasic Calcium Phosphate, Anhydrous, USP | 30.61 | 20.61 | 30.61 |
| (Fujicalin SG) | | | |
| Silicified High Density Microcrystalline Cellulose (Prosolv HD 90), USP | 30.00 | 40.00 | 15.00 |
| Methacrylic Acid Copolymer | 10.00 | 10.00 | 15.00 |
| (Eudragit L 100-55) | | | |
| Fumaric Acid | 5.00 | -- | 5.00 |
| Behenate, NF | 8.78 | 8.78 | 8.78 |
| (Compritol 888 ATO) | | | |
| **Total** | **100.0** | **100.0** | **100.0** |

The purpose of this example was to assess the bioavailability of three anagrelide HCl extended release tablet formulations Formulation 1 Formulation 2Formulation 3, compared to an immediate release anagrelide HCl formulation (Agrylin®, Lot No. RPA 0002A), following a 1 mg dose, and to determine the safety and tolerability of the extended release formulations in healthy volunteers.

The extent of absorption of each drug (AUCₒ₋ₜ) the maximum concentration of drug in plasma (Cₘₐₓ), and the time of the maximum concentration (Tₘₐₓ) were evaluated.

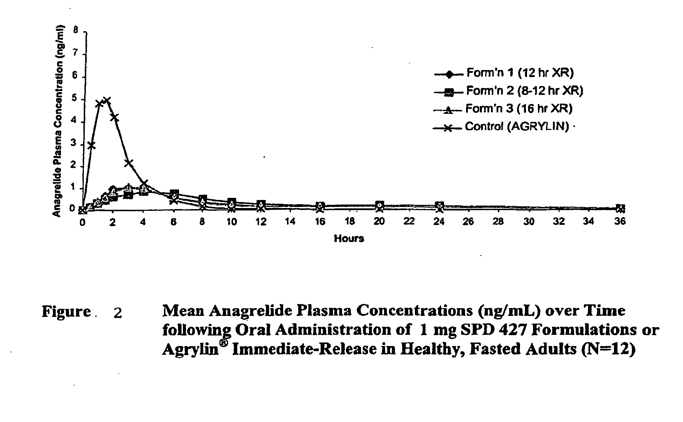

The mean plasma concentrations for anagrelide following administration of a single 1 mg dose of Agrylin®, or the anagrelide HCl extended release Formulations 1, 2, or 3, are given in Figure 2.

The corresponding pharmacokinetic parameters for each formulation are given in Table 8 below.

**Table 8 Mean Pharmacokinetic Parameters for Anagrelide**

| **Treatment** | **AUC_{0-inf} (ng.hr/mL)** | **AUC₀₋ₜ (ng.hr/mL)** | **Cₘₐₓ (ng/mL)** | **Tₘₐₓ (hr)** | **T_{width} ½Cₘₐₓ (hr)** |
|---|---|---|---|---|---|
| Formulation 1 | 9.24* | 8.47* | 1.15* | 3.41* | 5.06 |
| Formulation 2 | 10.32* | 9.10* | 1.01* | 3.17* | 5.99 |
| Formulation 3 | 9.41* | 8.48* | 1.17* | 3.46* | 4.30 |
| Agrylin® | 14.89 | 14.46 | 5.67 | 1.25 | 2.17 |

| **Bioequivalence** | | | | | |
|---|---|---|---|---|---|
| Formulation 1: Ratio of test-to-ref. | 0.61 | 0.58 | 0.21 | | |
| 90% CI | 0.49-0.76 | 0.46-0.73 | 0.17-0.27 | | |
| Formulation 2: Ratio of test-to-ref. | 0.59 | 0.53 | 0.17 | | |
| 90% CI | 0.47-0.74 | 0.42-0.67 | 0.14-0.22 | | |
| Formulation 3: Ratio of test-to-ref. | 0.66 | 0.64 | 0.25 | | |
| 90% CI | 0.52 | 0.50-0.81 | 0.20-0.32 | | |

| | | | | | |
|---|---|---|---|---|---|
| * p<0.05 compared to Agrylin® by Dunnett's test | | | | | |

All three experimental formulations exhibited extended release characteristics for drug delivery. The extent of absorption of anagrelide from the experimental formulations relative to immediate-release Agrylin^{®} (ratio of test-to-reference) was 58%, 53% and 64% for the Formulations 1, 2 and 3, respectively.

A measure of the extent of delay in release of active pharmaceutical ingredient of the experimental formulations was the time to reach maximum concentration (Tₘₐₓ) values observed for anagrelide. The Tₘₐₓ values were approximately 2 hours later than that observed for Agrylin^{®}, demonstrating about a 2.6-fold delay in time to reach Cₘₐₓ for the three extended release formulations compared with the immediate-release Agrylin^{®}. Furthermore, the length of time at which the anagrelide plasma concentration remained above ½Cₘₐₓ (T_{width} ½Cₘₐₓ) was approximately two- to three-fold greater for the extended release formulations than the immediate release formulation, demonstrating good extended release characteristics.

No serious adverse events were reported. One subject discontinued due to an adverse event (head cold) considered not related to study drug. There were a total of 46 adverse events (AEs) experienced by eleven (11) subjects. The most frequently reported events (>5%), included asthenia, headache, somnolence, and dizziness, and were not unexpected. These events have been reported frequently by the target patient population at large receiving Agrylin® for the treatment of thrombocythemia secondary to all myeloproliferative disorders. Treatment emergent AEs are summarized in Table 9.

**Table 9 Treatment Emergent AEs**

| **Preferred Term (Costart)** | **Count** | **% (n=12)** |
|---|---|---|
| Asthenia | 12 | 26.1 |
| Headache | 9 | 19.6 |
| Somnolence | 6 | 13.0 |
| Dizziness | 5 | 10.9 |
| Tachycardia | 2 | 4.3 |
| Infection | 2 | 4.3 |
| Nausea | 2 | 4.3 |
| Vomit | 2 | 4.3 |
| Pain Abd | 1 | 2.2 |
| Palpitations | 1 | 2.2 |
| Pain | 1 | 2.2 |
| Hem | 1 | 2.2 |
| Voice alteration | 1 | 2.2 |
| Thirst | 1 | 2.2 |
| **Total** | **46** | **100.0** |

The data would indicate that adverse events (AEs) observed in subjects following dosage with the extended release formulation were not as prevalent as AEs observed following the immediate release formulation (Agrylin^{®}). More than 50% of the reported AEs were observed following Agrylin^{®} administration. The number of adverse events following administration of the new extended release formulations were 8/46 (17%) for Formulation 1, 9/46 (20%) for Formulation 2, and 4/46 (9%) for Formulation 3 compared to 25/46 (54%) for the marketed Agrylin^{®}. The incidence of subjects reporting adverse events for the new extended-release formulations (4/12 (33%), 6/12 (50%), and 2/12 (17%) for Formulations 1, 2, and 3, respectively) also were substantially lower than the incidence observed in the Agrylin® group (10/12 (83%)). The number and percentage of subjects reporting adverse events are presented in Table 10.

**Table 10 Treatment Emergent Adverse Events per Treatment Group**

| **Drug Treatment** | **Preferred Term (Costart)** | **No. Subjects Reporting** | **% Subjects Reporting** | **No. AEs Reported** |
|---|---|---|---|---|
| Formulation 1 | Asthenia | 3 | 25.0 | 3 |
| (n=12) | Nausea | 1 | 8.3 | 1 |
| | Pain | 1 | 8.3 | 1 |
| | Somnolence | 1 | 8.3 | 1 |
| | Thirst | 1 | 8.3 | 1 |
| | Vomit | 1 | 8.3 | 1 |
| | | | | |
| Formulation 2 | Asthenia | 3 | 25.0 | 3 |
| (n=12) | Dizziness | 2 | 16.7 | 2 |
| | Headache | 2 | 16.7 | 2 |
| | Somnolence | 1 | 8.3 | 1 |
| | Hem | 1 | 8.3 | 1 |
| | | | | |
| Formulation 3 | Asthenia | 1 | 9.3 | 1 |
| (n=11) | Headache | 1 | 9.3 | 1 |
| | Infect | 1 | 9.3 | 1 |
| | Voice alteration | 1 | 9.3 | 1 |
| | | | | |
| Agrylin® | Headache | 6 | 50.0 | 6 |
| (n=12) | Asthenia | 5 | 41.7 | 5 |
| | Somnolence | 3 | 25.0 | 4 |
| | Dizziness | 3 | 25.0 | 3 |
| | Tacchycardia | 2 | 16.7 | 2 |
| | Nausea | 1 | 8.3 | 1 |
| | Pain Abdominal | 1 | 8.3 | 1 |
| | Infect | 1 | 8.3 | 1 |
| | Vomit | 1 | 8.3 | 1 |
| | Palpitations | 1 | 8.3 | 1 |
| | | | | |
| **Total (n=12)** | | | | 46 |

With respect to clinical laboratory and physical examination findings, statistically significant (p<0.01) increases in pulse compared with baseline were observed with Agrylin® at 2, 4, and 24 hours post dose. Significant increases were observed at 24 hours post dose for Formulations 1 and 3. Only Agrylin® showed a statistically significant changed from baseline for blood pressure (i.e. at 24 hours post dose for diastolic blood pressure).

## Claims

1. A sustained release pharmaceutical composition, in a form of a tablet comprising a blend of: (a) at least one pharmaceutically active agent that has a pH-dependent solubility; (b) at least one non-pH dependent sustained release agent; (c) at least one pH dependent agent that increases the rate of release of said at least one pharmaceutically active agent from the tablet at a pH in excess of 5.5 which includes at least one organic acid, maintaining an acidic microenvironment in the tablet, wherein said at least one pH dependent agent includes at least one enteric agent and wherein said at least one pharmaceutically active agent is selected from the group consisting of guanfacine, anagrelide, guanethidine monosulfate, guanadrel sulphate, reserpine, propranolol, metoprolol, atenolol, timolol, erythromycin, clonidine, chlorpheniramine, bromopheniramine, diltiazem, and scopolamine.

2. The composition of Claim 1 wherein said at least one pharmaceutically active agent is guanfacine.

3. The composition of Claim 1 wherein said at least one pharmaceutically active agent is guanfacine hydrochloride.

4. The composition of Claim 1 wherein said at least one pharmaceutically active agent is anagrelide.

5. The composition of Claim 1 wherein said at least one pharmaceutically active agent is anagrelide hydrochloride.

6. The composition of Claim 1 wherein said non-pH dependent sustained release agent is selected from the group consisting of ethylcellulose, cellulose acetate, vinyl acetate/vinyl chloride copolymers, acrylate/methacrylate copolymers, polyethylene oxide, hydroxypropyl methylcellulose, hydroxyethyl cellulose, hydroxypropyl cellulose, karaya gum, acacia gum, tragacanth gum, locust bean gum, guar gum, methyl cellulose, beeswax, carnauba wax, cetyl alcohol, hydrogenated vegetable oils, and stearyl alcohol.

7. The composition of Claim 1 wherein said at least one enteric agent is selected from the group consisting of cellulose acetate phthalate, hydroxypropyl methylcellulose phthalate, polyvinyl acetate phthalate, methacrylic acid copolymers, cellulose acetate trimellitate, hydroxypropyl methylcellulose acetate, succinate, shellac, and zein.

8. The composition of claim 1, wherein said organic acid is present in the composition in an amount of from 0.5 wt. % to 40 wt. %.

9. The composition of any one of the preceding Claims wherein said organic acid is selected from the group consisting of citric acid, fumaric acid, tartaric acid, adipic acid, glucono delta lactone, and malic acid.

10. The composition of claim 1 wherein said pH-dependent agent that increases the rate of release of the at least one pharmaceutically active agent from the tablet at a pH in excess of 5. 5 is an agent that maintains an acidic microenvironment in the composition.

11. The composition of claim 9 wherein said organic acid is fumaric acid.

12. The composition of any one of the preceding claims further comprising a binding agent.

13. The composition of claim 12 wherein said binding agent is selected from the group consisting of polyvinyl pyrrolidone, starch, methylcellulose, hydroxypropyl methylcellulose, carboxymethyl cellulose, sucrose solution, dextrose solution, acacia, tragacanth, and locust bean gum.

14. The composition of claim 13 wherein said binder is hydroxypropyl methylcellulose.

15. The composition of any one of the preceding claims wherein said pharmaceutically active agent is present in the composition in an amount of from 0.1 wt. % to 70 wt. %.

16. The composition of claim 15 wherein said pharmaceutically active agent is present in the composition in an amount of from 1 wt. % to 40 wt. %.

17. The composition of any one of the preceding claims wherein said non-pH-dependent sustained release agent is present in the composition in an amount of from 5 wt. % to 50 wt. %.

18. The composition of claim 17 wherein said non-pH-dependent sustained release agent is present in the composition in an amount of from 10 wt. % to 30 wt. %.

19. The composition of claim 1 wherein said pH-dependent agent is present in the composition in an amount of from 0.5 wt. % to 40 wt. %.

20. The composition of claim 19 wherein said pH-dependent agent is present in the composition in an amount of from 1 wt. % to 20 wt. %.

21. The composition of claim 6 wherein said non-pH-dependent sustained release agent is ethylcellulose.

22. The composition of claim 6 wherein said non-pH-dependent sustained release agent is hydroxypropyl methylcellulose.

23. The composition of claim 6 wherein said non-pH-dependent sustained release agent is an acrylate/methacrylate copolymer.

24. The composition of claim 6 wherein said non-pH-dependent sustained release agent is polyethylene oxide.

25. The composition of claim 7 wherein said at least one enteric agent is a methacrylic acid copolymer.

26. The composition of any one of the preceding claims further comprises a bulking agent.

27. The composition of claim 26 wherein said bulking agent is dibasic calcium phosphate.

28. The composition of claim 26 wherein said bulking agent is microcrystalline cellulose.

29. The composition of any one of the preceding claims further comprising a lubricant.

30. The composition of claim 29 wherein said lubricant is glyceryl behenate.

31. The composition of claim 29 wherein said lubricant is magnesium stearate.

32. Use of a composition of any one of the preceding claims for the manufacture of a pharmaceutical preparation for treating myeloproliferative blood disorders in a patient.

33. The use of claim 32 wherein said myeloproliferative blood disorder is essential thrombocythemia.

34. The use of claim 32 wherein said myeloproliferative blood disorder is chronic myelogenous leukemia.

35. The use of claim 32 wherein said myeloproliferative blood disorder is polycythemia vera.

36. The use of claim 32, wherein said myeloproliferative blood disorder is agnogenic myeloid metaplasia.

37. The use of claim 32 wherein the amount of the active agent anagrelide is 0.01 mg to 15 mg.

38. The use of claim 37 wherein the amount of the active agent anagrelide is 0.1 mg to 10 mg_{.}

39. The use of claim 38 wherein the amount of the active agent anagrelide is 0.1 mg to 5 mg.

40. The use of claim 39 wherein the amount of the active agent anagrelide is 0.5 mg to 2 mg.

41. The pharmaceutical composition of claim 1 comprising: (a) guanfacine; (b) Hydroxypropyl Methylcellulose (c) Ammonio Methacrylate Copolymer (d) microcrystalline cellulose; (e) a methacrylic acid copolymer; (f) glyceryl behenate; and (g) Fumaric Acid, (h) Lactose Monohydrate (i) Povidone; and (j) Crospovidone Granulated Blend.

42. The pharmaceutical composition of claim 1 comprising: (a) Guanfacine hydrochloride; (b) Hydroxypropyl Methylcellulose (c) Ammonio Methacrylate Copolymer (d) microcrystalline cellulose; (e) a methacrylic acid copolymer; (f) glyceryl behenate; and (g) Fumaric Acid, (h) Lactose Monohydrate (i) Povidone; and (j) Crospovidone Granulated Blend.

43. Use of the composition of any one of the preceding claims 1 to 31 or 41 or 42 for the manufacture of a pharmaceutical preparation for treating an attention deficit disorder or attention deficit with hyperactivity disorder in a patient.

## Patentansprüche

1. Pharmazeutische Zusammensetzung mit anhaltender Wirkstofffreisetzung in Form einer Tablette, umfassend eine Mischung von: (a) mindestens einem pharmazeutischen Wirkstoff mit pH-abhängiger Löslichkeit; (b) mindestens einem nicht-pH-abhängigen Mittel zur anhaltenden Wirkstofffreisetzung; (c) mindestens einem pH-abhängigen Mittel, das die Freisetzungsgeschwindigkeit des mindestens einen pharmazeutischen Wirkstoffes aus der Tablette bei einem pH-Wert über 5,5 erhöht, das mindestens eine organische Säure umfasst und eine saure Mikroumgebung in der Tablette aufrechterhält, wobei das mindestens eine pH-abhängige Mittel mindestens ein erst im Darm lösliches Mittel umfasst und wobei der mindestens eine pharmazeutische Wirkstoff aus der Gruppe ausgewählt ist, die besteht aus Guanfacin, Anagrelid, Guanethidinmonosulfat, Guanadrel-sulfat, Reserpin, Propranolol, Metoprolol, Atenolol, Timolol, Erythromycin, Clonidin, Chlorpheniramin, Brompheniramin, Diltiazem und Scopolamin.

2. Zusammensetzung nach Anspruch 1, wobei es sich beim mindestens einen pharmazeutischen Wirkstoff um Guanfacin handelt.

3. Zusammensetzung nach Anspruch 1, wobei es sich beim mindestens einen pharmazeutischen Wirkstoff um Guanfacin-hydrochlorid handelt.

4. Zusammensetzung nach Anspruch 1, wobei es sich beim mindestens einen pharmazeutischen Wirkstoff um Anagrelid handelt.

5. Zusammensetzung nach Anspruch 1, wobei es sich beim mindestens einen pharmazeutischen Wirkstoff um Anagrelid-hydrochlorid handelt.

6. Zusammensetzung nach Anspruch 1, wobei das nicht-pH-abhängige Mittel zur anhaltenden Wirkstofffreisetzung aus der Gruppe ausgewählt ist, die besteht aus Ethylcellulose, Celluloseacetat, Vinylacetat/Vinylchlorid-Copolymeren, Acrylat-Methacrylat-Copolymeren, Polyethylenoxid, Hydroxypropylmethylcellulose, Hydroxyethylcellulose, Hydroxypropylcellulose, Karayagummi, Gummi arabicum, Tragantgummi, Johannisbrotkernmehl, Guarkernmehl, Methylcellulose, Bienenwachs, Carnaubawachs, Cetylalkohol, hydrierten pflanzlichen Ölen und Stearylalkohol.

7. Zusammensetzung nach Anspruch 1, wobei das mindestens eine erst im Darm lösliche Mittel aus der Gruppe ausgewählt ist, die besteht aus Celluloseacetophthalat, Hydroxypropylmethylcellulosephthalat, Polyvinylacetophthalat, Methacrylsäure-Copolymeren, Celluloseacetotrimellitat, Hydroxypropylmethylcelluloseacetat, Succinat, Schellack und Zein.

8. Zusammensetzung nach Anspruch 1, wobei die organische Säure in der Zusammensetzung in einer Menge von 0,5 Gew.-% bis 40 Gew.-% vorliegt.

9. Zusammensetzung nach einem der vorstehenden Ansprüche, wobei die organische Säure aus der Gruppe ausgewählt ist, die besteht aus Citronensäure, Fumarsäure, Weinsäure, Adipinsäure, Glucono-delta-lacton und Äpfelsäure.

10. Zusammensetzung nach Anspruch 1, wobei das pH-abhängige Mittel, das die Freisetzungsgeschwindigkeit des mindestens einen pharmazeutischen Wirkstoffes aus der Tablette bei einem pH-Wert über 5,5 erhöht, ein Mittel ist, das eine saure Mikroumgebung in der Zusammensetzung aufrechterhält.

11. Zusammensetzung nach Anspruch 9, wobei es sich bei der organischen Säure um Fumarsäure handelt.

12. Zusammensetzung nach einem der vorstehenden Ansprüche, zusätzlich umfassend ein Bindemittel.

13. Zusammensetzung nach Anspruch 12, wobei das Bindemittel aus der Gruppe ausgewählt ist, die besteht aus Polyvinylpyrrolidon, Stärke, Methylcellulose, Hydroxypropylmethylcellulose, Carboxymethylcellulose, Saccharoselösung, Dextroselösung, Gummi arabicum, Tragant und Johannisbrotkernmehl.

14. Zusammensetzung nach Anspruch 13, wobei es sich beim Bindemittel um Hydroxypropylmethylcellulose handelt.

15. Zusammensetzung nach einem der vorstehenden Ansprüche, wobei der pharmazeutische Wirkstoff in der Zusammensetzung in einer Menge von 0,1 Gew.-% bis 70 Gew.-% vorliegt.

16. Zusammensetzung nach Anspruch 15, wobei der pharmazeutische Wirkstoff in der Zusammensetzung in einer Menge von 1 Gew.-% bis 40 Gew.-% vorliegt.

17. Zusammensetzung nach einem der vorstehenden Ansprüche, wobei das nicht-pH-abhängige Mittel zur anhaltenden Freisetzung in der Zusammensetzung in einer Menge von 5 Gew.-% bis 50 Gew.-% vorliegt.

18. Zusammensetzung nach Anspruch 17, wobei das nicht-pH-abhängige Mittel zur anhaltenden Freisetzung in der Zusammensetzung in einer Menge von 10 Gew.-% bis 30 Gew.-% vorliegt.

19. Zusammensetzung nach Anspruch 1, wobei das pH-abhängige Mittel in der Zusammensetzung in einer Menge von 0,5 Gew.-% bis 40 Gew.-% vorliegt.

20. Zusammensetzung nach Anspruch 19, wobei das pH-abhängige Mittel in der Zusammensetzung in einer Menge von 1 Gew.-% bis 20 Gew.-% vorliegt.

21. Zusammensetzung nach Anspruch 6, wobei es sich beim nicht-pH-abhängigen Mittel zur anhaltenden Freisetzung um Ethylcellulose handelt.

22. Zusammensetzung nach Anspruch 6, wobei es sich beim nicht-pH-abhängigen Mittel zur anhaltenden Freisetzung um Hydroxypropylmethylcellulose handelt.

23. Zusammensetzung nach Anspruch 6, wobei es sich beim nicht-pH-abhängigen Mittel zur anhaltenden Freisetzung um ein Acrylat/Methylacrylat-Copolymer handelt.

24. Zusammensetzung nach Anspruch 6, wobei es sich beim nicht-pH-abhängigen Mittel zur anhaltenden Freisetzung um Polyethylenoxid handelt.

25. Zusammensetzung nach Anspruch 7, wobei es sich bei dem mindestens einen erst im Darm löslichen Mittel um ein Methacrylsäure-Copolymer handelt.

26. Zusammensetzung nach einem der vorstehenden Ansprüche, zusätzlich umfassend einen Füllstoff.

27. Zusammensetzung nach Anspruch 26, wobei es sich beim Füllstoff um dibasisches Calciumphosphat handelt.

28. Zusammensetzung nach Anspruch 26, wobei es sich beim Füllstoff um mikrokristalline Cellulose handelt.

29. Zusammensetzung nach einem der vorstehenden Ansprüche, zusätzlich umfassend ein Gleitmittel.

30. Zusammensetzung nach Anspruch 29, wobei es sich beim Gleitmittel um Glycerylbehenat handelt.

31. Zusammensetzung nach Anspruch 29, wobei es sich beim Gleitmittel um Magnesiumstearat handelt.

32. Verwendung einer Zusammensetzung nach einem der vorstehenden Ansprüche zur Herstellung eines pharmazeutischen Präparats zur Behandlung von myeloproliferativen Blutstörungen bei einem Patienten.

33. Verwendung nach Anspruch 32, wobei es sich bei der myeloproliferativen Blutstörung um essentielle Thrombozytämie handelt.

34. Verwendung nach Anspruch 32, wobei es sich bei der myeloproliferativen Blutstörung um chronische myelogene Leukämie handelt.

35. Verwendung nach Anspruch 32, wobei es sich bei der myeloproliferativen Blutstörung um Polycythaemia vera handelt.

36. Verwendung nach Anspruch 32, wobei es sich bei der myeloproliferativen Blutstörung um agnogene myeloische Metaplasie handelt.

37. Verwendung nach Anspruch 32, wobei die Menge des Wirkstoffes Anagrelid 0,01 mg bis 15 mg beträgt.

38. Verwendung nach Anspruch 37, wobei die Menge des Wirkstoffes Anagrelid 0,1 mg bis 10 mg beträgt.

39. Verwendung nach Anspruch 38, wobei die Menge des Wirkstoffes Anagrelid 0,1 mg bis 5 mg beträgt.

40. Verwendung nach Anspruch 39, wobei die Menge des Wirkstoffes Anagrelid 0,5 mg bis 2 mg beträgt.

41. Pharmazeutische Zusammensetzung nach Anspruch 1, umfassend: (a) Guanfacin, (b) Hydroxypropylmethylcellulose, (c) Ammoniomethacrylat-Copolymer, (d) mikrokristalline Cellulose, (e) ein Methacrylsäure-Copolymer, (f) Glycerylbehenat und (g) Fumarsäure, (h) Lactose-monohydrat, (i) Povidon und (j) Crospovidon-Granulatgemisch.

42. Pharmazeutische Zusammensetzung nach Anspruch 1, umfassend: (a) Guanfacin-hydrochlorid, (b) Hydroxypropylmethylcellulose, (c) Ammoniomethacrylat-Copolymer, (d) mikrokristalline Cellulose, (e) ein Methacrylsäure-Copolymer, (f) Glycerylbehenat und (g) Fumarsäure, (h) Lactose-monohydrat, (i) Povidon und (j) Crospovidon-Granulatgemisch.

43. Verwendung der Zusammensetzung nach einem der vorstehenden Ansprüche 1 bis 31 oder 41 oder 42 zur Herstellung eines pharmazeutischen Präparats zur Behandlung einer Aufmerksamkeitsdefizitstörung oder einer Aufmerksamkeitsdefizitstörung mit Hyperaktivitätsstörung bei einem Patienten.

## Revendications

1. Composition pharmaceutique à libération prolongée sous la forme d'un comprimé, comprenant un mélange de : (a) au moins un agent ayant une activité pharmaceutique qui a une solubilité dépendante du pH ; (b) au moins un agent de libération prolongée non dépendant du pH ; (c) au moins un agent dépendant du pH qui augmente la vitesse de libération dudit au moins un agent ayant une activité pharmaceutique à partir du comprimé à un pH dépassant 5,5, qui comprend au moins un acide organique, en maintenant un microenvironnement acide dans le comprimé, dans laquelle l'au moins un agent dépendant du pH comprend au moins un agent entérique, et dans laquelle ledit au moins un agent ayant une activité pharmaceutique est choisi dans le groupe constitué par la guanfacine, l'anagrélide, le monosulfate de guanéthidine, le sulfate de guanadrel, la réserpine, le propranolol, le métoprolol, l'aténolol, le timolol, l'érythromycine, la clonidine, la chlorphéniramine, la bromophéniramine, le diltiazem, et la scopolamine.

2. Composition selon la revendication 1, dans laquelle ledit au moins un agent ayant une activité pharmaceutique est la guanfacine.

3. Composition selon la revendication 1, dans laquelle ledit au moins un agent ayant une activité pharmaceutique est le chlorhydrate de guanfacine.

4. Composition selon la revendication 1, dans laquelle ledit au moins un agent ayant une activité pharmaceutique est l'anagrélide.

5. Composition selon la revendication 1, dans laquelle ledit au moins un agent ayant une activité pharmaceutique est le chlorhydrate d'anagrélide.

6. Composition selon la revendication 1, dans laquelle ledit agent de libération prolongée non dépendant du pH est choisi dans le groupe constitué par l'éthylcellulose, l'acétate de cellulose, les copolymères d'acétate de vinyle/chlorure de vinyle, les copolymères d'acrylate/méthacrylate, le poly(oxyde d'éthylène), l'hydroxypropylméthylcellulose, l'hydroxyéthylcellulose, l'hydroxypropylcellulose, la gomme karaya, la gomme arabique, la gomme adragante, la gomme de caroube, la gomme de guar, la méthylcellulose, la cire d'abeilles, la cire de carnauba, l'alcool cétylique, les huiles végétales hydrogénées, et l'alcool stéarylique.

7. Composition selon la revendication 1, dans laquelle ledit au moins un agent entérique est choisi dans le groupe constitué par l'acétophtalate de cellulose, le phtalate d'hydroxypropylméthylcellulose, le poly(acétophtalate de vinyle), les copolymères d'acide méthacrylique, l'acétotrimellitate de cellulose, l'acétate d'hydroxypropylméthylcellulose, le succinate, la gomme laque, et la zéine.

8. Composition selon la revendication 1, dans laquelle ledit acide organique est présent dans la composition en une quantité de 0,5 % en poids à 40 % en poids.

9. Composition selon l'une quelconque des revendications précédentes, dans laquelle ledit acide organique est choisi dans le groupe constitué par l'acide citrique, l'acide fumarique, l'acide tartrique, l'acide adipique, la glucono-5-lactone, et l'acide malique.

10. Composition selon la revendication 1, dans laquelle ledit agent dépendant du pH qui augmente la vitesse de libération de l'au moins un agent ayant une activité pharmaceutique à partir du comprimé à un pH dépassant 5,5 est un agent qui maintient un microenvironnement acide dans la composition.

11. Composition selon la revendication 9, dans lequel ledit acide organique est l'acide fumarique.

12. Composition selon l'une quelconque des revendications précédentes, comprenant en outre un agent liant.

13. Composition selon la revendication 12, dans laquelle ledit agent liant est choisi dans le groupe constitué par la polyvinylpyrrolidone, l'amidon, la méthylcellulose, l'hydroxypropylméthylcellulose, la carboxyméthylcellulose, le saccharose en solution, le dextrose en solution, la gomme arabique, la gomme adragante, et la gomme de caroube.

14. Composition selon la revendication 13, dans laquelle ledit liant est l'hydroxypropylméthylcellulose.

15. Composition selon l'une quelconque des revendications précédentes, dans laquelle ledit agent ayant une activité pharmaceutique est présent dans la composition en une quantité de 0,1 % en poids à 70 % en poids.

16. Composition selon la revendication 15, dans laquelle ledit agent ayant une activité pharmaceutique est présent dans la composition en une quantité de 1 % en poids à 40 % en poids.

17. Composition selon l'une quelconque des revendications précédentes, dans laquelle ledit agent de libération prolongée non dépendant du pH est présent dans la composition en une quantité de 5 % en poids à 50 % en poids.

18. Composition selon la revendication 17, dans laquelle ledit agent de libération prolongée non dépendant du pH est présent dans la composition en une quantité de 10 % en poids à 30 % en poids.

19. Composition selon la revendication 1, dans laquelle ledit agent dépendant du pH est présent dans la composition en une quantité de 0,5 % en poids à 40 % en poids.

20. Composition selon la revendication 19, dans laquelle ledit agent dépendant du pH est présent dans la composition en une quantité de 1 % en poids à 20 % en poids.

21. Composition selon la revendication 6, dans laquelle ledit agent de libération prolongée non dépendant du pH est l'éthylcellulose.

22. Composition selon la revendication 6, dans laquelle ledit agent de libération prolongée non dépendant du pH est l'hydroxypropylméthylcellulose.

23. Composition selon la revendication 6, dans laquelle ledit agent de libération prolongée non dépendant du pH est un copolymère d'acrylate/méthacrylate.

24. Composition selon la revendication 6, dans laquelle ledit agent de libération prolongée non dépendant du pH est le poly(oxyde d'éthylène).

25. Composition selon la revendication 7, dans laquelle ledit au moins un agent entérique est un copolymère d'acide méthacrylique.

26. Composition selon l'une quelconque des revendications précédentes, comprenant en outre un agent de remplissage.

27. Composition selon la revendication 26, dans laquelle ledit agent de remplissage est le phosphate de calcium dibasique.

28. Composition selon la revendication 26, dans laquelle ledit agent de remplissage est la cellulose microcristalline.

29. Composition selon l'une quelconque des revendications précédentes, comprenant en outre un lubrifiant.

30. Composition selon la revendication 29, dans laquelle ledit lubrifiant est le béhénate de glycéryle.

31. Composition selon la revendication 29, dans laquelle ledit lubrifiant est le stéarate de magnésium.

32. Utilisation d'une composition selon l'une quelconque des revendications précédentes pour la fabrication d'une préparation pharmaceutique destinée au traitement de troubles sanguins myéloprolifératifs chez un patient.

33. Utilisation selon la revendication 32, dans laquelle ledit trouble sanguin myéloprolifératif est une thrombocythénie essentielle.

34. Utilisation selon la revendication 32, dans laquelle ledit trouble sanguin myéloprolifératif est une leucémie myéloïde chronique.

35. Utilisation selon la revendication 32, dans laquelle ledit trouble sanguin myéloprolifératif est une érythrémie.

36. Utilisation selon la revendication 32, dans laquelle ledit trouble sanguin myéloprolifératif est une métaplasie myéloïde angiogénique.

37. Utilisation selon la revendication 32, dans laquelle la quantité de l'agent actif anagrélide est de 0,01 mg à 15 mg.

38. Utilisation selon la revendication 37, dans laquelle la quantité de l'agent actif anagrélide est de 0,1 mg à 10 mg.

39. Utilisation selon la revendication 38, dans laquelle la quantité de l'agent actif anagrélide est de 0,1 mg à 5 mg.

40. Utilisation selon la revendication 39, dans laquelle la quantité de l'agent actif anagrélide est de 0,5 mg à 2 mg.

41. Composition pharmaceutique selon la revendication 1 comprenant : (a) de la guanfacine ; (b) de l'hydroxypropylméthylcellulose ; (c) un copolymère d'ammonio-méthacrylate ; (d) de la cellulose microcristalline ; (e) un copolymère d'acide méthacrylique ; (f) du béhénate de glycéryle ; et (g) de l'acide fumarique ; (h) du lactose monohydraté ; (i) de la povidone ; et (j) de la crospovidone en mélange granulé.

42. Composition pharmaceutique selon la revendication 1 comprenant : (a) du chlorhydrate de guanfacine ; (b) de l'hydroxypropylméthylcellulose ; (c) un copolymère d'ammonio-méthacrylate ; (d) de la cellulose microcristalline ; (e) un copolymère d'acide méthacrylique ; (f) du béhénate de glycéryle ; et (g) de l'acide fumarique ; (h) du lactose monohydraté ; (i) de la povidone ; et (j) de la crospovidone en mélange granulé.

43. Utilisation de la composition selon l'une quelconque des revendications 1 à 31, 41 et 42, pour la fabrication d'une préparation pharmaceutique destinée à traiter un trouble déficitaire de l'attention ou un trouble déficitaire de l'attention avec hyperactivité chez un patient.
